# EUROPEAN PATENT SPECIFICATION

(11) **EP 0 860 147 B1**
(45) Date of publication and mention of the grant of the patent: **17.05.2006**
(21) Application number: 98102799.8
(22) Date of filing: 18.02.1998
(51) Int. Cl.: A61B 17/00, A61F 2/38

(54) **Knee prosthesis**
Knieprothese
Prothèse de genou

(30) Priority: 21.02.1997 IT MI970386
(43) Date of publication of application: 26.08.1998
(73) Proprietor: Patella, Vittorio, 70125 Bari (IT)
(72) Inventor: Patella, Vittorio, 70125 Bari (IT)
(74) Representative: Cicogna, Franco

(56) References cited:
- EP-A- 0 333 642
- EP-A- 0 538 895
- CH-A- 664 686
- FR-A- 2 698 536
- FR-A- 2 736 819
- FR-A- 2 755 004
- GB-A- 1 534 263

## Description

### BACKGROUND OF THE INVENTION

The present invention relates to an improved knee prosthesis, which has been specifically designed for providing optimum bone anchoring and articulation mobility properties.

A very important problem affecting the making of knee prostheses is that of properly affixing the prosthesis, upon having applied it.

The fixing of the prosthesis is at first obtained by providing a geometrical congruency between the prosthesis and the bone; however, such a geometrical congruency, even if it is well designed, is not sufficient to provide the necessary strength of the fixation.

Accordingly, the mentioned anchoring properties are conventionally enhanced by additional methods, such as: a cementation between the bone and prosthesis, the provision of surfaces having a morphology suitable for the bone regrowth, the use of locking screws, the application of bone regrowth promoting coatings, these methods being conventionally used in a mutual combination.

A further problem of difficult solution is the quick wear of mutually coupled prosthesis components due to a not perfect kinematic congruency of commercially available prosthesis systems during a mutual contact movement between the prosthesis femoral component and the tibial component thereof.

This movement is a combined rolling and sliding movement.

The latter represents a main cause of a premature wear of the mutually contacting components, which wear increases with the increasing of the coupling forces of the components, and which frequently requires the removal of the overall prosthesis.

The document CH-664686 discloses a knee prosthesis construction substantially according to the preamble of claim 1.

### SUMMARY OF THE INVENTION

Accordingly, the aim of the present invention is to overcome the above mentioned problems, by providing a knee prosthesis specifically designed for an optimum bone anchoring and for meeting a kinematic congruency of the prosthesis system, and, more specifically, in the action thereof accompanying the mutual contact movement between the femoral condyles and the tibial flat part.

Within the scope of the above mentioned aim, a main object of the present invention is to provide such a prosthesis able of suitably exploiting the bone regrowth phenomenon in order to provide an optimum anchoring of said prosthesis.

Another object of the present invention is to provide such a knee prosthesis including a kinematic mechanism specifically designed for reproducing the articulation of a human knee and resisting against the stresses, both of static and dynamic nature, the prosthesis is subjected to during its use.

Yet another object of the present invention is to provide such a knee prosthesis which can effectively replace damaged knee articulations thereby allowing a full recovering of the mobility function of a patient.

According to the present invention, the above mentioned aim and objects, as well as yet other objects, which will become more apparent hereinafter, are achieved by a knee prosthesis construction, according to claim 1.

### BRIEF DESCRIPTION OF THE DRAWINGS

Further characteristics and advantages of the knee prosthesis construction according to the present invention, will become more apparent hereinafter from the following detailed disclosure of a preferred, though not exclusive, embodiment of said knee prosthesis construction, which is illustrated, by way of an indicative, but not limitative, example, in the accompanying drawings, where:
Figure 1 is an exploded perspective view illustrating the prosthesis according to the present invention;
Figure 2 is a side elevation view illustrating a detail of the subject prosthesis, and clearly showing the coupling between the femoral component and the tibial component thereof;
Figures 3 and 4 are further elevation views illustrating two possible tibial components of a knee prosthesis. Figure 3 and 4 are not part of the invention and are only included for elucidation purposes.
Figure 5 is a top plan view illustrating the tibial component of the prosthesis according to the invention;
Figure 6 is a schematic view illustrating a knee prosthesis after the application thereof; Figure 6 is also not part of the invention and is included for elucidation purposes
Figure 7 is a side perspective view illustrating a tibial stub element having, according to the invention an omega configuration, and provided with a plurality of throughgoing holes suitable to improve the anchoring of the subject prosthesis; and
Figure 8 is a bottom view of the tibial component of the subject prosthesis, illustrated in Figure 7.

### DESCRIPTION OF THE PREFERRED EMBODIMENTS

With reference to the number reference of the above mentioned figures, the prosthesis according to the present invention, which has been generally indicated by the reference number 1, comprises a patellar component, of a per se known type and not specifically shown for simplicity, a femoral component 2 as well as a tibial component 3.

The femoral component 2, in particular, is preferably made of a biocompatible metal material or any other biocompatible materials depending on requirements, and has a convex configuration on the side thereof provided for coupling with the tibial component 3, and being concave on its inner side, which is provided for coupling with the femur 4.

The tibial component 3 is provided, on the side thereof provided for coupling the the femoral component 2, with a pair of preferably spherical concavities 5a and 5b therewith is engaged the convex side of the femoral component 2, having a corresponding configuration.

The patellar component, the femoral component 2 and tibial component 3 comprise, even individually, at least an endomedular stub element 6a, 6b, 7, 8 and 9a, 9b of hollow configuration and provided with transverse openings or holes in order to provide an optimum anchoring to the bone as the latter regrowths, the bone regrowth occurring both inside and outside of said stub element, with a consequent formation of a mechanical stable bone grid.

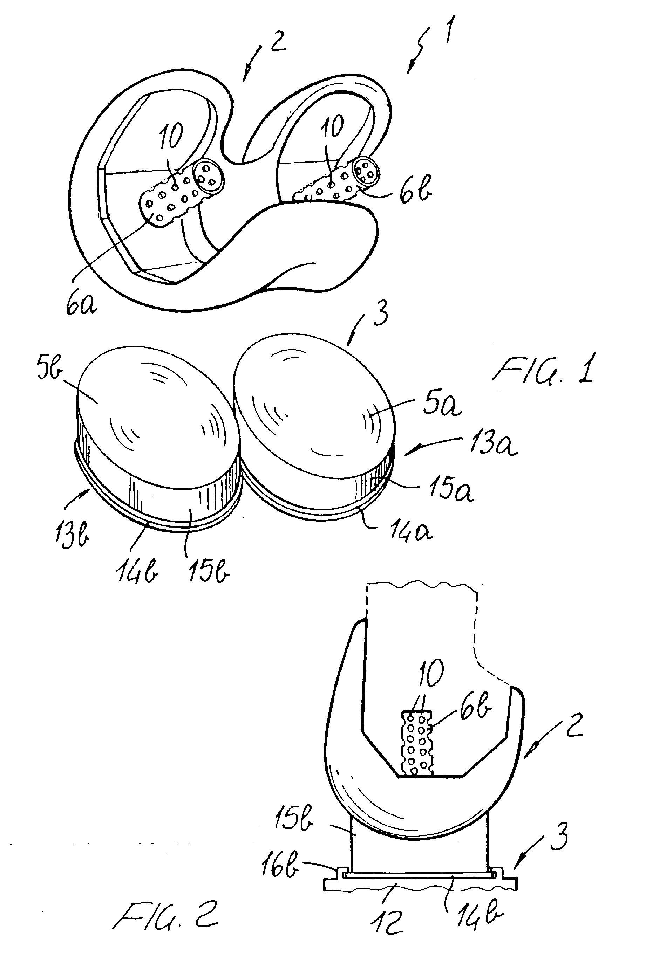

More specifically, as is shown in Figure 1, the femoral component 2 is provided with two stub elements 6a and 6b, which also extend on the concave side thereof and which are provided, in their cross sections, with a circular configuration.

In this connection it should be apparent that the configuration of the stub elements 6a and 6b can also be different from that disclosed, depending on requirements.

The free end portions of the mentioned stub elements 6a and 6b are provided with a lead-in edge in order to limit a possible damaging to the bone as the stub elements are engaged therein.

As shown, through the mentioned stub elements 6a and 6b a plurality of transverse openings or throughgoing holes 10 are formed, the edges of said openings being suitably rounded in order to prevent microcracks from forming, which would inevitably cause a breaking of the bone bridge being formed.

The mentioned transverse openings 10 can have any suitable configurations, from a series of small holes to a single broad opening, with a series of possible intermediate solutions, to be adopted depending on specific requirements and designed for promoting as far as possible a quick and stable bone regrowth.

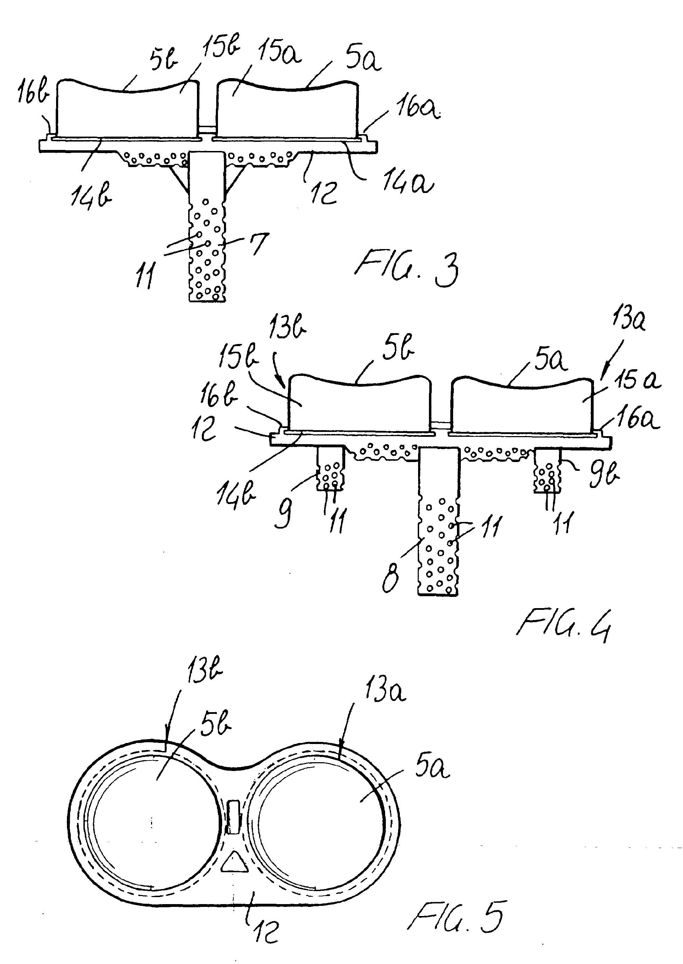

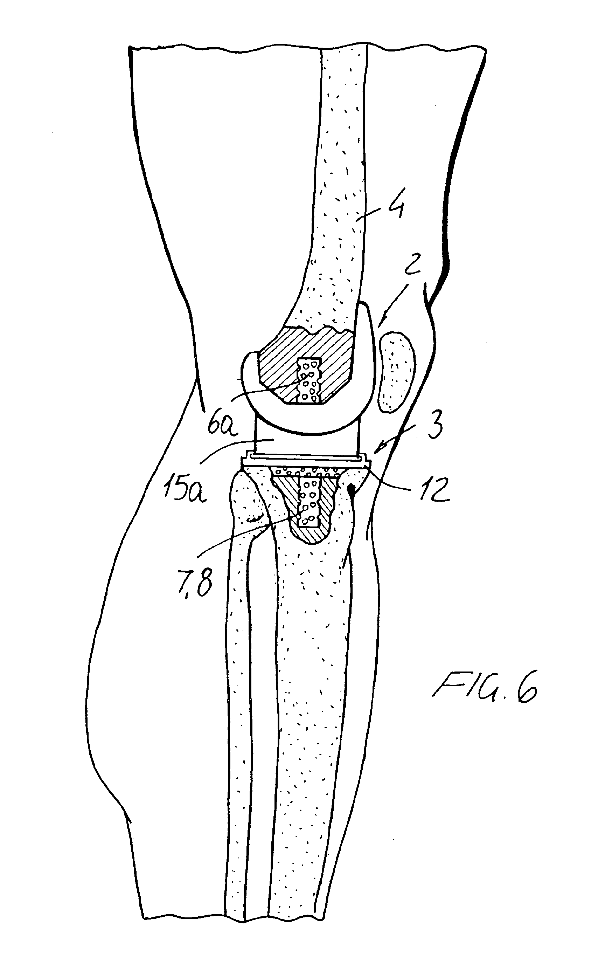

The stub elements 7, 8, 9a and 9b of the tibial component 3 as shown in Figures 3, 4 and 6 are made likewise to the stub elements 6a and 6b of the disclosed femoral component.

The stub elements 7, 8, 9a and 9b are also provided with transverse openings 11, which can have different configurations, depending on requirements, and the edges thereof are suitably rounded, for the above disclosed reasons.

The free end portions of the stub elements 7, 8, 9a and 9b are also provided with respective lead-in edges in order to prevent the bone from being damaged as the prosthesis is applied.

The tibial component 3 is provided with a flat element 12, preferably made of a biocompatible metal material, which defines at the top thereof a sliding plane, on which bear two components 13a and 13b including a metal base 14a and 14b provided for slidably engaging with the flat element 12 and above which a top insert of a biocompatible synthetic material 15a and 15b is provided, said top insert being preferably made of polyethylene, in which the concavities 5a and 5b for the femoral component 2 are defined.

Preferably, the components 13a and 13b have a substantially disc-like configuration, either circular or elliptical, or they can have any other suitable configuration, depending on requirements.

The mutually contacting surfaces of the flat element 12 and of the two components 13a and 13b are made smooth in order to facilitate the sliding of the two components 13a and 13b on the flat element 12.

Moreover, each of said components 13a and 13b is held in a respective recess 16a and 16b, of corresponding configuration, and formed on the top face of the flat element or portion 12.

More specifically, the metal base 14a, 14b is provided with a perimetrical edge, which is held inside the recess 16a and 16b by a corresponding cutout edge portion, delimiting the recesses or seats 16a and 16b.

The subject knee prosthesis is engaged in its seat by impact, after having resected the end portions of the bones, so as to provide a geometrical coupling with the configurations of the prosthesis components.

During this operating step, the stub elements 7, 8, 9a and 9b, entering the bone, are filled in their inside by the bone tissue and, then, as promoted by the starting mechanical stability, a further biologic stability will be obtained for the growth of the transverse bone bridges through the openings 10, 11 which will transform the bone encompassing the stub element into a bone grid, suitable to resist against the outer stresses the prosthesis is subjected to.

The specifically designed coupling between the components 13a and 13b and flat element or portion 12 will provide the possibility of splitting the sliding component through two pairs of surfaces: the articulation surfaces proper and those on the tibial flat portion.

Thus, the components 15a and 15b, which are made of a biocompatible synthetic material, preferably of polyethylene, will be subjected to a less wear, thereby providing a greater duration of the implant.

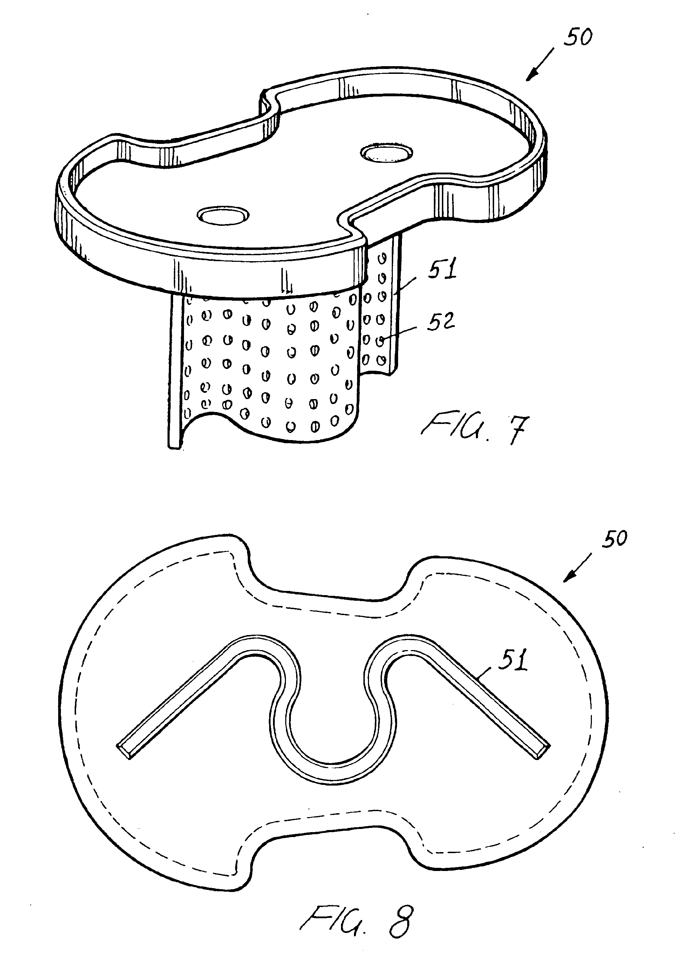

With reference to the tibial component of the prosthesis according to the present invention, as shown in Figures 7 and 8, it should be pointed out that this component is characterized by a specifically designed tibial stub element 50, which is provided with an omega configuration or shape.

That same tibial stub element 50 is moreover provided, at its omega-shape anchoring surface 51, with a plurality of small holes 52, adapted to improve and accelerate the bone fixation of said stub element 50.

Moreover, a less restraining against a rotary movement on the transverse articulation plane is obtained, thereby promoting a natural extra-rotation movement of the tibial flat portion, during the deambulation; this will also involve a more accurate operation of the articular ligaments thereby further improving the prothesic stability of the overall novel articulation.

Thus, the above disclosed prosthesis construction will allow a homogeneous distribution of the loads and will provide a suitable articular kinematic, thereby providing a perfect stability of time duration.

Moreover, owing to its specifically designed features, the subject prosthesis construction is very reliable and safe in operation.

From the above disclosure it should be apparent that the invention fully achieves the intended aim and objects.

In particular, the fact is to be pointed out that a knee prosthesis has been provided which affords a very high overall mechanical stability and kinematic congruency of the articulation surfaces, thereby providing its components with improved performance and duration properties.

In practicing the invention, the used materials, provided that they are compatible to the intended application, as well as the contingent size and shapes, can be any, depending on requirements.

## Claims

1. A knee prosthesis construction (1) for providing an optimum mutual coupling between the contacting prothesing tibial and femoral articulating surfaces during the deambulation comprising a tibial component (3) and two components (13a, 13b),
- said tibial component (3) having a flat portion (12) defining at the top thereof a sliding plane on which said two components (13a, 13b) can be supported,
- said components (13a, 13b) being provided on a side opposite to said sliding plane, with a spherical cap concavity (5a, 5b), for coupling with a femoral component (2), the femoral component (2) being suitable to be connected to the bottom end of the femur, and having a corresponding configuration,
**characterized in that** said tibial component (3) of said prosthesis is provided with an omega-shaped tibial stub element (50).

2. A knee prosthesis construction, according to Claim 1, **characterized in that** the two components (13a, 13b) of said tibial component (3) comprise a biocompatible synthetic material top insert (15a, 15b) in which said spherical cap concavities (5a, 5b) are formed.

3. A knee prosthesis construction, according to Claim 1, **characterized in that** the two components (13a, 13b) of said tibial component have a substantially disc-like configuration.

4. A knee prosthesis construction, according to Claim 1, **characterized in that** said flat portion (12) and said two components (13a, 13b) define mutually smooth contacting surfaces for facilitating the sliding of said two components (13a, 13b) on said flat portion (12).

5. A knee prosthesis construction, according to Claim 1, **characterized in that** said two components (13a, 13b) have a base which is held in a correspondingly shaped recess defined on the top face of said flat portion (12).

6. A knee prosthesis construction, according to Claim 1, **characterized in that** said tibial stub element (50) is provided, at an omega-shaped bottom portion (51) thereof, with a plurality of small holes (52) suitable to improve the bone anchoring of said tibial stub element to a patient body.

7. A knee prosthesis construction, according to Claim 1, further comprising femoral (2) and patellar components including each a hollow endomedullar stub (6a, 6b, 7, 8; 9a, 9b), **characterized in that** said hollow endomedullar stubs are provided with transverse openings (10) for anchoring said hollow stubs to a bone, during the regrowth of said bone, both inside and outside of said endomedullar stubs.

8. A knee prosthesis construction, according to Claim 7, **characterized in that** said hollow endomedullar stubs (7, 8; 9a, 9b) comprise each a free end portion, with a lead-in edge to prevent a bone from being damaged as said prosthesis is engaged therein.

9. A knee prosthesis construction, according to Claim 7, **characterized in that** said transverse openings (10) are provided with rounded edges.

## Patentansprüche

1. Knieprothesenkonstruktion (1) zum Bereitstellen einer optimalen gegenseitigen Kopplung zwischen den sich berührenden, die Prothese bildenden tibialen und femoralen, ein Gelenk bildenden Oberflächen während des Gehens, welche eine tibiale Komponente (3) und zwei Komponenten (13a, 13b) umfasst,
- wobei die tibiale Komponente (3) einen flachen Abschnitt (12) aufweist, der an deren Oberseite eine gleitende Ebene definiert, auf der die zwei Komponenten (13a, 13b) gelagert werden können,
- wobei die Komponenten (13a, 13b) auf einer Seite, die der gleitenden Ebene gegenüberliegt, bereitgestellt sind, und eine kugelförmige Kappenhöhlung (5a, 5b) zur Kopplung mit einer femoralen Komponente (2) aufweisen, wobei die femorale Komponente (2) sich dazu eignet, mit dem unteren Ende des Femur verbunden zu werden, und einen entsprechenden Aufbau aufweist,
**dadurch gekennzeichnet, dass** die tibiale Komponente (3) der Prothese mit einem omegaförmigen tibialen Stumpfelement (50) versehen ist.

2. Knieprothesenkonstruktion nach Anspruch 1, **dadurch gekennzeichnet, dass** die zwei Komponenten (13a, 13b) der tibialen Komponente (3) einen oberen Einsatz (15a, 15b) aus biokompatiblem synthetischem Material umfassen, in dem die kugelförmigen Kappenhöhlungen (5a, 5b) gebildet sind.

3. Knieprothesenkonstruktion nach Anspruch 1, **dadurch gekennzeichnet, dass** die zwei Komponenten (13a, 13b) der tibialen Komponente einen im Wesentlichen scheibenähnlichen Aufbau aufweisen.

4. Knieprothesenkonstruktion nach Anspruch 1, **dadurch gekennzeichnet, dass** der flache Abschnitt (12) und die zwei Komponenten (13a, 13b) gegenseitig weiche, sich berührende Oberflächen definieren, um das Gleiten der zwei Komponenten (13a, 13b) auf dem flachen Abschnitt (12) zu erleichtern.

5. Knieprothesenkonstruktion nach Anspruch 1, **dadurch gekennzeichnet, dass** die zwei Komponenten (13a, 13b) eine Basis aufweisen, die in einer entsprechend geformten Vertiefung, die auf der oberen Fläche des flachen Abschnitts (12) definiert ist, gehalten wird.

6. Knieprothesenkonstruktion nach Anspruch 1, **dadurch gekennzeichnet, dass** das tibiale Stumpfelement (50) an einem omegaförmigen unteren Abschnitt (51) von diesem mit mehreren kleinen Löchern (52) versehen ist, die sich dafür eignen, die Knochenverankerung des tibialen Stumpfelements an den Körper eines Patienten zu verbessern.

7. Knieprothesenkonstruktion nach Anspruch 1, die weiterhin femorale (2) und patellare Komponenten umfasst, die jeweils einen hohlen endomedullären Stumpf (6a, 6b, 7, 8; 9a, 9b) einschließen, **dadurch gekennzeichnet, dass** die hohlen endomedullären Stümpfe mit quer laufenden Öffnungen (10) versehen sind, um die hohlen Stümpfe während dem Nachwachsen des Knochens, sowohl im Inneren als auch am Äußeren der endomedullären Stümpfe, an einen Knochen zu verankern.

8. Knieprothesenkonstruktion nach Anspruch 7, **dadurch gekennzeichnet, dass** jeder der hohlen endomedullären Stümpfe (7, 8; 9a, 9b) einen freien Endabschnitt mit einem Einführ-Rand aufweist, um zu verhindern, dass ein Knochen beschädigt wird, wenn die Prothese darin eingesetzt wird.

9. Knieprothesenkonstruktion nach Anspruch 7, **dadurch gekennzeichnet, dass** die quer laufenden Öffnungen (10) mit abgerundeten Rändern versehen sind.

## Revendications

1. Conception de prothèse de genou (1) permettant un couplage mutuel optimal entre les surfaces tibiales en contact avec la prothèse et celles de l'articulation fémorale pendant la marche, comprenant un composant tibial (3) et deux composants (13a, 13b),
- ledit composant tibial (3) ayant une partie plate (12) délimitant au niveau de sa partie supérieure un plan de glissement sur lequel lesdits composants (13a, 13b) peuvent être supportés.
- lesdits composants (13a, 13b) étant disposés sur un côté opposé audit plan de glissement, avec une calotte sphérique concave (5a, 5b) pour le couplage avec un composant fémoral (2), ce composant fémoral (2) étant destiné à être relié à la partie inférieure du fémur et présentant une forme correspondante,
**caractérisée en ce que** ledit composant tibial (3) de ladite prothèse est muni d'une embase tibiale en forme d'oméga (50).

2. Conception de prothèse de genou selon la revendication 1, **caractérisée en ce que** les deux composants (13a, 13b) dudit composant tibial (3) comprennent un insert supérieur en matériau synthétique biocompatible (15a, 15b) dans lequel sont formées lesdites calottes sphériques concaves (5a, 5b).

3. Conception de prothèse de genou selon la revendication 1, **caractérisée en ce que** les deux composants (13a, 13b) dudit composant tibial (3) présentent sensiblement une forme de disque.

4. Conception de prothèse de genou selon la revendication 1, **caractérisée en ce que** ladite partie plate (12) et lesdits composants (13a, 13b) délimitent des surfaces lisses de contact mutuel pour faciliter le glissement desdits composants (13a, 13b) sur ladite partie plate (12).

5. Conception de prothèse de genou selon la revendication 1, **caractérisée en ce que** lesdits composants (13a, 13b) présentent une base maintenue dans un creux de forme correspondante aménagé sur la face supérieure de ladite partie plate (12).

6. Conception de prothèse de genou selon la revendication 1, **caractérisée en ce que** ladite embase tibiale (50) est munie, au niveau de sa partie inférieure en forme d'oméga (51), d'une pluralité de petits trous (52) qui servent à améliorer la fixation de l'os de ladite embase tibiale dans le corps d'un patient.

7. Conception de prothèse de genou selon la revendication 1, comprenant en outre un composant fémoral (2) et un composant rotulien comprenant chacun une embase endomédullaire creuse (6a, 6b, 7, 8 ; 9a, 9b), **caractérisée en ce que** lesdites embases endomédullaires creuses sont munies d'ouvertures transversales (10), pour la fixation desdites embases creuses à un os pendant la croissance de celui-ci, à l'intérieur et à l'extérieur desdites embases endomédullaires.

8. Conception de prothèse de genou selon la revendication 7, **caractérisée en ce que** lesdites embases endomédullaires creuses (7, 8 ; 9a, 9b) comprennent chacune une partie libre avec un bord de guidage d'entrée pour empêcher l'endommagement d'un os lors de l'insertion de ladite prothèse.

9. Conception de prothèse de genou selon la revendication 7, **caractérisée en ce que** lesdites ouvertures transversales (10) sont munies de bords arrondis.
